# EUROPÄISCHE PATENTSCHRIFT

(11) **EP 2 273 958 B1**
(45) Veröffentlichungstag und Bekanntmachung des Hinweises auf die Patenterteilung: **27.08.2014**
(21) Anmeldenummer: 09745433.4
(22) Anmeldetag: 31.03.2009
(51) Int. Cl.: A61F 9/007

(54) **HOHLNADEL FÜR EIN AUGENCHIRURGISCHES INSTRUMENT**
HOLLOW NEEDLE FOR AN OPHTHALMIC SURGICAL INSTRUMENT
AIGUILLE CREUSE POUR UN INSTRUMENT DE CHIRURGIE OCULAIRE

(30) Priorität: 16.05.2008 DE 102008023967
(43) Veröffentlichungstag der Anmeldung: 19.01.2011
(73) Patentinhaber: Geuder AG, 69126 Heidelberg (DE)
(72) Erfinder: GEUDER, Volker, 69126 Heidelberg (DE)
(74) Vertreter: Ullrich & Naumann
(86) Internationale Anmeldenummer: PCT/DE2009/000407
(87) Internationale Veröffentlichungsnummer: WO 2009/138049

(56) Entgegenhaltungen:
- EP-A- 0 421 285
- WO-A-2007/119107
- US-A- 4 869 715
- US-A- 5 464 389
- US-A- 5 562 609
- US-A- 6 159 175
- US-A1- 2003 004 455
- US-B1- 6 592 541

## Beschreibung

Die Erfindung betrifft eine Hohlnadel für ein augenchirurgisches Instrument zur in-vivo-Zertrümmerung organischer Linsen mittels Ultraschall, mit einem Anschlussbereich zum Ankoppeln an das Instrument und einem am freien Ende ausgebildeten Arbeitsbereich mit einer Wirkfläche zum Abstrahlen von Ultraschallwellen, wobei sich durch die Hohlnadel hindurch ein im Arbeitsbereich offener Absaugkanal zum Absaugen von Linsentrümmern erstreckt, dessen Öffnung durch die Wirkfläche gebildet bzw. begrenzt ist.

Eine gattungsbildende Hohlnadel ist aus der DE 196 46 881 C1 bekannt. Im Konkreten handelt es sich dabei um eine Hohlnadel für ein augenchirurgisches Instrument zur in-vivo-Zertrümmerung von Linsen durch Hochfrequenzbetätigung der Hohlnadel, wobei die Hohlnadel gleichzeitig zum Absaugen von Linsen durch einen inneren Absaugkanal dient. Die Hohlnadel umfasst ein ringförmig ausgebildetes Stirnende, das die Öffnung des Absaugkanals bildet.

Ultraschallbetätigte Hohlnadeln der gattungsbildenden Art werden bei Kataraktoperationen in der Augenchirurgie verwendet. Das freie Ende der Hohlnadel wird in eine hochfrequente Axialbewegung versetzt und unmittelbar an den Katarakt herangeführt. Vom ringförmigen Stirnende werden Ultraschallwellen zur Emulsifikation des Gewebes abgestrahlt. Abgetrennte Linsenteile bzw. Linsentrümmer werden durch die Hohlnadel hindurch gemeinsam mit einer dem Auge zugeführten Spühlflüssigkeit abgeführt.

Zur Verstärkung des emittierten Ultraschallfeldes ist es bereits bekannt, das stirnseitige bzw. freie Ende der Hohlnadel, d. h. die dortige Wirkfläche, gezahnt auszugestalten, um nämlich die Wirkfläche durch die Zahnung zu vergrößern. Die zum Abstrahlen von Ultraschallwellen dienende Wirkfläche wird dadurch vergrößert, so dass die Effizienz des Instruments bzw. der Hohlnadel verbessert ist.

Die aus der DE 196 46 881 C1 bekannte Hohlnadel ist in der Praxis jedoch problematisch, da nicht selten größere Linsentrümmer abgesaugt werden, die im Bereich der Absaugöffnung zu einer zumindest momentanen Verstopfung führen. Außerdem besteht die Gefahr, dass der Operateur mit der Öffnung des Absaugkanals zu nah in den Bereich des nicht abzusaugenden Materials gelangt, so dass dort aufgrund eines entstehenden zu großen Unterdrucks eine Beschädigung des Gewebes eintreten kann. Würde man den Saugdruck im augenchirurgischen Instrument erhöhen, könnte man zwar einem ungewollte Verstopfung im vorderen Bereich des Absaugkanals entgegenwirken, würde jedoch weiterreichende Beschädigungen bzw. Verletzungen in Kauf nehmen.

Des Weiteren sei auf die Druckschrift US 6 159 175 hingewiesen.

Der vorliegenden Erfindung liegt daher die Aufgabe zugrunde, eine Hohlnadel für ein augenchirurgisches Instrument der gattungsbildenden Art derart auszugestalten und weiterzubilden, dass unter Vermeidung ungewollter Verletzungen eine Verstopfung im vorderen Bereich des Saugkanals wirksam vermieden ist.

Die voranstehende Aufgabe ist erfindungsgemäß durch eine Hohlnadel für ein augenchirurgisches Instrument zur in-vivo-Zertrümmerung organischer Linden mittels Ultraschall mit den Merkmalen des Patentanspruchs 1 gelöst. Danach ist die gattungsbildende Hohlnadel dadurch gekennzeichnet, dass der Absaugkanal mindestens eine als Bypass wirkende weitere Öffnung aufweist, die im Arbeitsbereich ausgebildet ist.

Erfindungsgemäß ist erkannt worden, dass sich eine Verstopfung im Arbeitsbereich der Hohlnadel durch eine einfache konstruktive Maßnahme vermeiden lässt, nämlich durch Ausbildung von Bezug auf den Strömungspfad als Bypass zu verstehenden weiteren Öffnungen im Arbeitsbereich. Mit anderen Worten sind im Arbeitsbereich neben der vorderen Ansaugöffnung weitere Öffnungen vorgesehen, durch die im Falle eines Ansaugens großer Linsentrümmer Luft angesaugt werden kann, so dass aufgrund des im Absaugkanal entstehenden Unterdrucks ein weiteres Hineinsaugen des Linsentrümmers und somit eine weiterreichende Verstopfung vermieden wird. Geht man davon aus, dass die meisten augenchirurgischen Instrumente pulsierende Unterdrücke und zumindest auch kurzzeitig Überdrücke im Absaugkanal erzeugen können, lässt sich ein nicht komplett in die Hohlnadel eingesaugter Linsentrümmer mühelos wieder ausstoßen, was im Falle einer bereits erfolgten Verstopfung nicht oder nicht mehr der Fall ist. Jedenfalls vermeiden die weiteren Öffnungen einen all zu großen Unterdruck im Absaugkanal, was bereits per se einer Verstopfung des Absaugkanals entgegen wirkt.

In vorteilhafter Weise lassen sich mehrere weitere Öffnungen entlang dem Umfang des Arbeitsbereichs vorsehen, wobei es von ganz besonderem Vorteil ist, dass die weiteren Öffnungen nahe der Wirkfläche ausgebildet sind. Die Ausgestaltung der weiteren Öffnungen nahe der Wirkfläche ist insoweit von ganz besonderem Vorteil, als genau dort einer ungewollten Verstopfung des Absaugkanals entgegengewirkt wird, wobei im weiteren Verlaufe des Absaugkanals der zum Abführen der zerkleinerten Linsentrümmer erforderliche Unterdruck aufgebaut werden kann. Wären die weiteren Öffnungen weiter entfernt von der Wirkfläche ausgebildet, wäre die absaugende Wirkung der Hohlnadel erheblich reduziert. Auch dies in erfindungsgemäßer Weise wirksam vermieden.

Die weiteren Öffnungen münden in die Arbeitsfläche, öffnen sich somit an der Arbeitsfläche nach außen. Eine solche Vorkehrung hat den Vorteil, dass durch diese Maßnahme nicht nur eine Art Bypass geschaffen ist, sondern auch die Arbeitsfläche ganz erheblich vergrößert ist, was wiederum die Emission von Ultraschallwellen bzw. das Ultraschallfeld begünstigt.

Die weiteren Öffnungen sind jeweils als Durchgang, insbesondere als Fräsung, Ausklinkung, usw. in der Wandung des Arbeitsbereichs der Hohlnadel ausgebildet. Eine lasertechnische Herstellung des Durchgangs ist ebenfalls denkbar und insbesondere angesichts der miniaturisierten Ausgestaltung des Arbeitsbereichs von Vorteil. Auch elektroerosive Methoden zur Herstellung des Durchgangs sind denkbar.

Der jeweilige Durchgang kann jedwede Form haben. In vorteilhafter Weise ist er als Schlitz in der Wandung des Arbeitsbereichs der Hohlnadel ausgebildet. Er kann eckig, oval, rund oder anderweitig ausgeführt sein. Wesentlich ist jedenfalls, dass er in seiner Größe bzw. in seiner Fläche so bemessen ist, dass ungeachtet des Durchgangs ein hinreichend großer Unterdruck im Arbeitsbereich der Hohlnadel entsteht, jedoch ein Festsaugen übergroßer Linsentrümmer wirksam vermieden ist.

In weiter vorteilhafter Weise ist der Arbeitsbereich gegenüber dem Hauptkörper der Hohlnadel radial erweitert. Mit anderen Worten weist die Hohlnadel einen radial erweiterten Arbeitsbereich auf, der vorzugsweise zylindrisch ausgebildet ist. Somit bildet der Arbeitsbereich der Hohlnadel eine Art Saugglocke, die sich am Ende des Arbeitsbereichs auf den eigentlichen Innendurchmesser der Hohlnadel bzw. auf den Durchmesser des Absaugkanals reduziert. Zwischen dem Arbeitsbereich und dem reduzierten Absaugkanal kann eine oder können mehrere Stufen vorgesehen sein, die aufgrund einer scharfkantigen oder gar gezackten Ausbildung ebenfalls den Zerkleinerungsvorgangs im Innern des Arbeitsbereichs begünstigen.

In weiter vorteilhafter Weise endet der Arbeitsbereich mit einer abgeschrägten Fläche, die die Wirkfläche bildet und deren vorderes Ende als Spitze der Hohlnadel zu verstehen ist. Beliebige Winkel der abgeschrägten Fläche zur Längsachse sind realisierbar, um nämlich auch dadurch die wirksame Fläche zu vergrößern.

Grundsätzlich ist es denkbar, dass die Wirkfläche in einer Ebene, vorzugsweise glatt, ausgebildet ist. Auch ist es denkbar, dass die Wirkfläche gewellt ausgebildet ist, wodurch die wirksame Fläche ebenfalls vergrößert ist.

In ganz besonders vorteilhafter Weise umfasst die Wirkfläche äquidistant zueinander ausgebildete Einschnitte bzw. Einkerbungen, die die eigentliche Wirkfläche entlang ihrem Umfange unterbrechen. Entsprechend einer solchen Ausgestaltung besteht die eigentliche Wirkfläche aus einzelnen Segmenten, die bspw. dreieckig oder mehreckig ausgeführt sein können.

Die Einkerbungen sind derart ausgebildet, dass der vordere Bereich der Außenwandung gegenüber der eigentlichen Wirkfläche mehrfach geöffnet ist, so dass dadurch mehrere weitere Öffnungen gebildet sind, die die eigentliche Wirkfläche unterbrechen. Mit anderen Worten ist die Außenwandung des Arbeitsbereichs - nach außen hin - unterbrochen, so dass im Falle des Näherns oder gar Aufsetzens der eigentlichen Wirkfläche eine Notventilation durch die seitlichen Öffnungen erfolgt. Durch diese Maßnahme ist die Entstehung eines zu hohen Unterdrucks im Inneren des Arbeitsbereichs wirksam vermieden.

In Bezug auf die Fertigung der Hohlnadel ist es von Vorteil, diese einstückig auszubilden, wobei diese aus Titan oder einer Titanlegierung bestehen kann.
- Fig. 1: in einer schematischen Ansicht ein erstes Ausführungsbeispiel einer erfindungsgemäßen Hohlnadel mit einer weiteren Öffnung im Arbeitsbereich,
- Fig. 2: in einer schematischen Ansicht, vergrößert, den Arbeitsbereich der Hohlnadel aus Fig. 1,
- Fig. 3: in einer schematischen Ansicht ein zweites Ausführungsbeispiel einer erfindungsgemäßen Hohlnadel mit mehreren weiteren Öffnungen im Sinne eines gezackten und nach außen geöffneten Arbeitsbereichs, und
- Fig. 4: in einer schematischen Ansicht, vergrößert, den Arbeitsbereich der Hohlnadel aus Fig. 3.

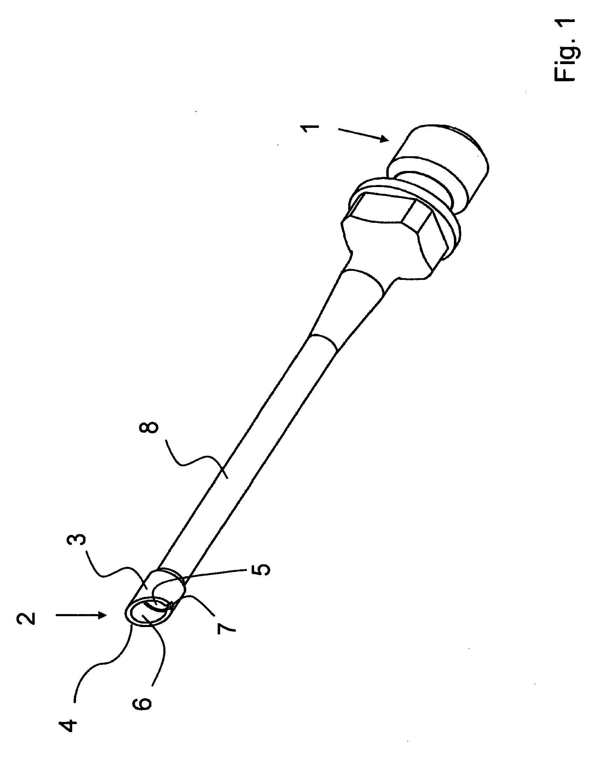

Fig. 1 zeigt eine Hohlnadel für ein augenchirurgisches Instrument zur in-vivo-Zertrümmerung organischer Linsen mittels Ultraschall. Die Hohlnadel umfasst einen Anschlussbereich 1 zum Ankoppeln an das in den Figuren nicht gezeigte Instrument und einen am freien Ende 2 ausgebildeten Arbeitsbereich 3. Der Arbeitsbereich 3 zum Abstrahlen von Ultraschallwellen, wobei sich durch die Hohlnadel hindurch ein im Arbeitsbereich 3 offener Absaugkanal 5 zum Absaugen von Linsentrümmern erstreckt. Die Öffnung 6 des Absaugkanals 5 ist durch die Wirkfläche 4 gebildet bzw. begrenzt.

Der Ansaugkanal 5 weist mindestens eine als Bypass wirkende weitere Öffnung 7 auf, die im Arbeitsbereich 3 ausgebildet ist.

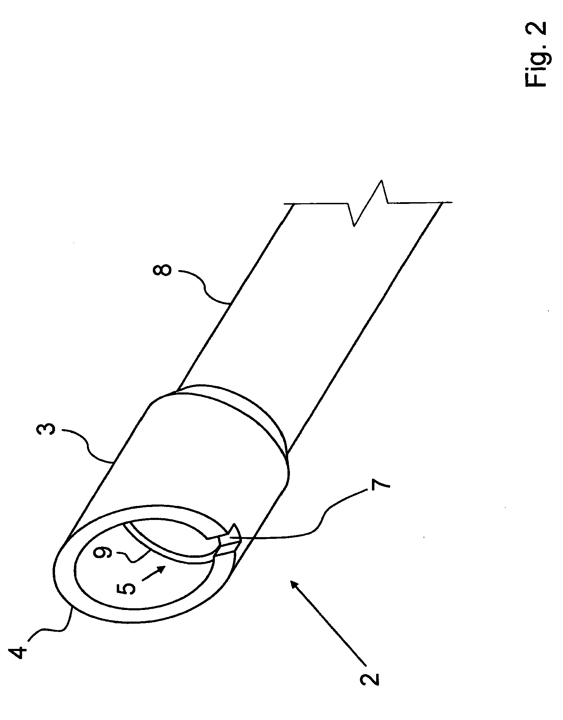

Bei der in den Fig. 1 und 2 gezeigten Hohlnadel ist eine einzige weitere Öffnung 7 vorgesehen, wobei in vorteilhafter Weise auch mehrere solcher Öffnungen 7 vorgesehen sein können.

Die weitere Öffnung 7 ist nahe der Wirkfläche 4 ausgebildet, nämlich als rechteckiger Durchgang. Die weitere Öffnung 7 endet in der Wirkfläche 4 und ist somit zur Wirkfläche 4 hin offen. Aufgrund dieser Maßnahme vergrößert die weitere Öffnung 7 die Wirkfläche 4 erheblich. Die Fig. 1 und 2 zeigen des Weiteren, dass der Arbeitsbereich 3 gegenüber dem Hauptkörper 8 der Nadel radial erweitert ist, nämlich zu einem radial erweiterten zylindrischen Arbeitsbereich 3. Das freie Ende des Arbeitsbereichs 3 ist abgeschrägt und durch die Wirkfläche 4 definiert.

Die Fig. 1 und 2 zeigen des Weiteren deutlich, dass im Inneren des Arbeitsbereichs 3 eine Stufe 9 oder zumindest ein abgeschrägter Übergangsbereich ausgebildet ist, der die Zerkleinerung des Linsenmaterials abermals begünstigt.

Des Weiteren sei angemerkt, dass sowohl die Außenkante als auch die Innenkante des Arbeitsbereichs 3, d. h. die innere und äußere Begrenzung der Wirkfläche 4, scharfkantig ausgeführt sein kann. Eine Fasung sowohl außen als auch innen würde die Wirkfläche 4 der Abstrahlung von Ultraschallwellen abermals begünstigen.

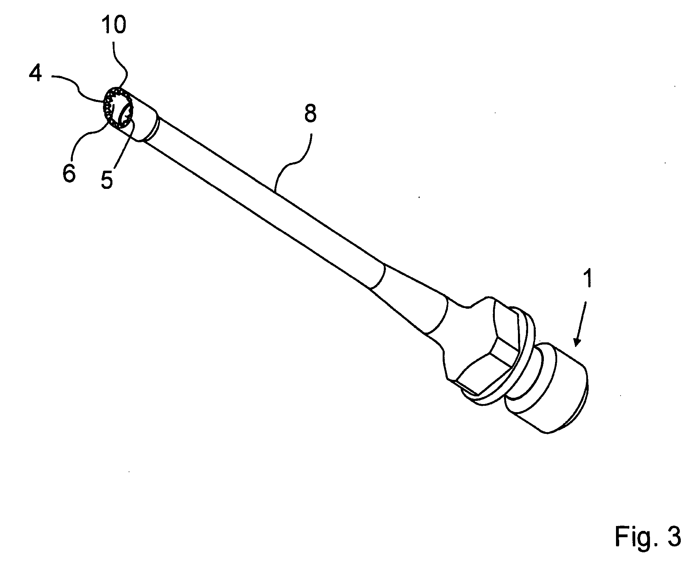

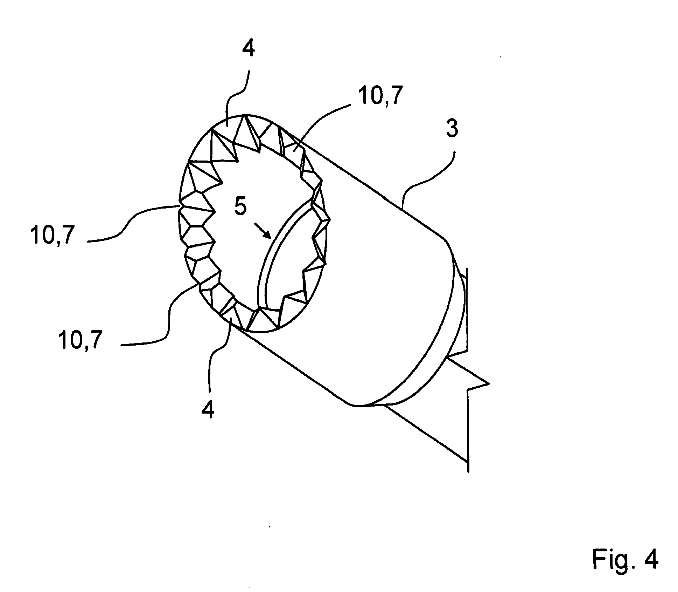

Bei dem in den Fig. 3 und 4 gezeigten Ausführungsbeispiel ist die Wirkfläche 4 durch äquidistante Einkerbungen 10 unterbrochen, wobei die Einkerbungen 10 auch den vorderen Bereich der Außenfläche des Arbeitsbereichs 3 unterbrechen bzw. schlitzen. Durch diese Maßnahme sind mehrere weitere Öffnungen 7 entlang dem Umfang des radial erweiterten Arbeitsbereichs 3 geschaffen. Gleichzeitig ist nach innen hin die Wirkfläche vergrößert, nämlich durch die Einkerbungen 10.

In Bezug auf Merkmale, die sich den Abbildungen nicht entnehmen lassen, sei zur Vermeidung von Wiederholungen auf den allgemeinen Teil der Beschreibung verwiesen.

Schließlich sei ausdrücklich darauf hingewiesen, dass das voranstehend beschriebene Ausführungsbeispiel der erfindungsgemäßen Hohlnadel lediglich zur Erörterung der beanspruchten Lehre dient, diese jedoch nicht auf das gezeigte Ausführungsbeispiele einschränkt.

### Bezugszeichenliste

- 1: Anschlussbereich
- 2: freies Ende
- 3: Arbeitsbereich
- 4: Wirkfläche
- 5: Absaugkanal
- 6: Öffnung (Ansaugöffnung)
- 7: weitere Öffnung (Bypass)
- 8: Hauptkörper der Hohlnadel
- 9: Stufe (im Innern des Arbeitsbereichs)
- 10: Einkerbungen (weitere Öffnung)

## Patentansprüche

1. Hohlnadel für ein augenchirurgisches Instrument zur In-vivo-Zertrümmerung organischer Linsen mittels Ultraschall, mit einem Anschlussbereich (1) zum Ankoppeln an das Instrument und einem am freien Ende (2) ausgebildeten Arbeitsbereich (3) mit einer stirnseitigen Wirkfläche (4) zum Abstrahlen von Ultraschallwellen,
wobei sich durch die Hohlnadel hindurch ein im Arbeitsbereich (3) offener Absaugkanal (5) zum Absaugen von Linsentrümmern erstreckt, dessen Öffnung (6) durch die Wirkfläche (4) gebildet bzw. begrenzt ist,
wobei die Wirkfläche (4) äquidistant ausgebildete Einkerbungen (10) aufweist, welche die wirksame Fläche der Wirkfläche (4) nach innen hin vergrößern und welche derart ausgebildet sind, dass der distale Bereich der Außenwandung des Arbeitsbereichs (3) gegenüber der Wirkfläche (4) mehrfach geöffnet ist,
wobei der Arbeitsbereich (3) Durchgänge (7) aufweist, welche die Wirkfläche (4) unterbrechen und für den Absaugkanal (5) als Bypass wirken,
wobei die Einkerbungen (10) die Wirkfläche (4) entlang des Umfangs derart unterbrechen, dass die Durchgänge (7) gebildet sind,
a) wobei einzelne dreieckige Segmente der Wirkfläche (4) gebildet werden,
oder
b) wobei die Einkerbungen (10) durch V-förmige Rillen gebildet sind, und wobei dreieckige oder mehreckige einzelne Segmente der Wirkfläche (4) gebildet werden.

2. Hohlnadel nach Anspruch 1, wobei die Durchgänge (7) als Fräsung und/oder Schlitz in der Wandung des Arbeitsbereichs (3) der Nadel ausgebildet sind.

3. Hohlnadel nach Anspruch 1 oder 2, wobei der Arbeitsbereich (3) gegenüber dem Hauptkörper (8) der Nadel radial erweitert ist.

4. Hohlnadel nach Anspruch 3, wobei der Arbeitsbereich (3) zylindrisch ausgebildet ist.

5. Hohlnadel nach einem der Ansprüche 1 bis 4, wobei der Arbeitsbereich (3) mit einer abgeschrägten Fläche endet, die die Wirkfläche (4) bildet und deren vorderes Ende die Spitze bildet.

6. Hohlnadel nach einem der Ansprüche 1 bis 5, wobei die Wirkfläche (4) gewellt ausgebildet ist.

7. Hohlnadel nach einem der Ansprüche 1 bis 6, wobei die Durchgänge (7) und die dreieckigen Segmente der Wirkfläche (4) alternierend angeordnet sind, oder wobei die aufeinanderfolgenden einzelnen Segmente der Wirkfläche (4) durch einen der Durchgänge (7) voneinander getrennt sind.

8. Hohlnadel nach einem der Ansprüche 1 bis 7, wobei die Durchgänge (7) V-förmig oder eckig ausgebildet sind.

9. Hohlnadel nach einem der Ansprüche 1 bis 8, wobei die Durchgänge (7) in dem Absaugkanal (5) münden und dadurch für den Absaugkanal (5) als Bypass wirken.

## Claims

1. Hollow needle for an ophthalmic surgical instrument for in-vivo fragmentation of organic lenses using ultrasound, having a connection region (1) for coupling to the instrument and an operating region (3) which is formed at the free end (2) and which has a front-side active face (4) for radiating ultrasound waves,
wherein there extends through the hollow needle a suction channel (5) which is open in the operating region (3) for suction of lens debris and whose opening (6) is formed or delimited by the active face (4),
wherein the active face (4) has indentations (10) which are constructed in an equidistant manner and which increase the effective surface-area of the active face (4) in an inward direction and which are constructed in such a manner that the distal region of the outer wall of the operating region (3) is open several times with respect to the active face (4),
wherein the operating region (3) has apertures (7) which interrupt the active face (4) and which act as a bypass for the suction channel (5),
wherein the indentations (10) interrupt the active face (4) along the periphery in such a manner that the apertures (7) are formed,
a) wherein individual triangular segments of the active face (4) are formed
or
b) wherein the indentations (10) are formed by V-shaped grooves and triangular or polygonal individual segments of the active face (4) are formed.

2. Hollow needle according to claim 1, wherein the apertures (7) are constructed as a milled portion and/or slot in the wall of the operating region (3) of the needle.

3. Hollow needle according to claim 1 or claim 2, wherein the operating region (3) is radially expanded with respect to the main body (8) of the needle.

4. Hollow needle according to claim 3, wherein the operating region (3) is constructed in a cylindrical manner.

5. Hollow needle according to any one of claims 1 to 4, wherein the operating region (3) terminates with a chamfered face which forms the active face (4) and whose front end forms the tip.

6. Hollow needle according to any one of claims 1 to 5, wherein the active face (4) is constructed in an undulating manner.

7. Hollow needle according to any one of claims 1 to 6, wherein the apertures (7) and the triangular segments of the active face (4) are arranged in an alternating manner, or wherein the sequential individual segments of the active face (4) are separated from each other by one of the apertures (7).

8. Hollow needle according to any one of claims 1 to 7, wherein the apertures (7) are constructed in a V-shaped or angular manner.

9. Hollow needle according to any one of claims 1 to 8, wherein the apertures (7) open in the suction channel (5) and thereby act as a bypass for the suction channel (5).

## Revendications

1. Aiguille creuse pour un instrument de chirurgie ophtalmologique permettant la fragmentation in vivo de lentilles organiques par ultrasons, comportant une zone de raccordement (1) pour le raccordement à l'instrument et une zone de travail (3), réalisée à l'extrémité libre (2) et munie d'une face active (4) frontale pour émettre les ondes ultrasonores,
un conduit d'aspiration (5) destiné à aspirer les fragments de lentille s'étendant à travers l'aiguille creuse, lequel est ouvert dans la zone de travail (3) et dont l'ouverture (6) est formée ou délimitée par la face active (4),
ladite face active (4) comportant des encoches (10) équidistantes, qui agrandissent vers l'intérieur la surface active de la face active (4) et qui sont réalisées de telle sorte que la zone distale de la paroi extérieure de la zone de travail (3) est ouverte plusieurs fois par rapport à la face active (4);
ladite zone de travail (3) comportant des passages (7) qui interrompent la face active (4) et agissent comme by-pass pour le conduit d'aspiration (5),
lesdites encoches (10) interrompant la face active (4) le long de son pourtour de manière à former les passages (7),
a) des segments triangulaires individuels de la face active (4) étant formés,
ou
b) les encoches (10) étant formées par des rainures en forme de V, et des segments triangulaires ou polygonaux individuels de la face active (4) étant formés.

2. Aiguille creuse selon la revendication 1, dans laquelle les passages (7) sont réalisés sous forme de fraisages et/ou de fentes dans la paroi de la zone de travail (3) de l'aiguille.

3. Aiguille creuse selon la revendication 1 ou 2, dans laquelle la zone de travail (3) est élargie radialement par rapport au corps principal (8) de l'aiguille.

4. Aiguille creuse selon la revendication 3, dans laquelle la zone de travail (3) est cylindrique.

5. Aiguille creuse selon l'une quelconque des revendications 1 à 4, dans laquelle la zone de travail (3) se termine par une surface en biseau, qui forme la face active (4) et forme la pointe de l'extrémité avant de cette dernière.

6. Aiguille creuse selon l'une quelconque des revendications 1 à 5, dans laquelle la face active (4) est réalisée sous forme ondulée.

7. Aiguille creuse selon l'une quelconque des revendications 1 à 6, dans laquelle les passages (7) et les segments triangulaires de la face active (4) sont disposés en alternance, ou dans laquelle les segments individuels successifs de la face active (4) sont séparés les uns des autres par l'un des passages (7).

8. Aiguille creuse selon l'une quelconque des revendications 1 à 7, dans laquelle les passages (7) sont réalisés en forme de V ou sous forme anguleuse.

9. Aiguille creuse selon l'une quelconque des revendications 1 à 8, dans laquelle les passages (7) débouchent dans le conduit d'aspiration (5) et, de ce fait, agissent comme by-pass pour le conduit d'aspiration (5).
